**Europäisches Patentamt**

**European Patent Office**

**Office européen des brevets**

(19)

(11) Publication number : **0 370 611 B1**

# EUROPEAN PATENT SPECIFICATION

(12)

(45) Date of publication of patent specification :
**17.06.92 Bulletin 92/25**

(51) Int. Cl.⁵ : **F25J 3/02, B01D 5/00, C07C 7/04**

(21) Application number : **89310356.4**

(22) Date of filing : **10.10.89**

(54) **Ethane recovery system.**

(30) Priority : **21.11.88 US 274243**

(43) Date of publication of application :
**30.05.90 Bulletin 90/22**

(45) Publication of the grant of the patent :
**17.06.92 Bulletin 92/25**

(84) Designated Contracting States :
**DE ES FR GB IT NL**

(56) References cited :
**FR-A- 2 399 396**
**US-A- 4 687 499**
**US-A- 4 851 020**
**US-A- 4 889 545**

(73) Proprietor : **MCDERMOTT INTERNATIONAL, INC.**
**1010 Common Street PO Box 60035**
**New Orleans Louisiana 70160 (US)**

(72) Inventor : **Montgomery, George Joseph, IV**
**1218 Timbergrove Drive**
**Houston Texas 77008 (US)**

(74) Representative : **Purvis, William Michael Cameron et al**
**D. Young & Co. 10 Staple Inn**
**London WC1V 7RD (GB)**

EP 0 370 611 B1

Jouve, 18, rue Saint-Denis, 75001 PARIS

## Description

The invention pertains to a method of separating ethane, from a natural gas stream.

In processes for separating hydrocarbon gas constituents, if the process utilizes a demethanizer then methane and other more volatile components are separated from the ethane and less volatile components of the gas stream. Should, however, the process utilize a de-ethanizer, then ethane and more volatile components (including methane) are separated from propane and less volatile components of the stream. In either case, the "recovered" components are in the column bottoms product while the "rejected" components are in the overhead vapour stream.

Many patents relating to this technology exist with each describing a unique method or improvement. Their emphasis, however, is generally directed to the processing of the hydrocarbon gas before it enters a demethanizer, de-ethanizer or other similar structure. Seldom is there emphasis on the processing of the overhead vapour stream after it leaves the demethanizer, etc. (for example US-A-4,453,958 and US-A-4,464,190 to Gulsby). However, some of those that do process the overhead vapour stream, such as US-A-4,171,964 and US-A-4,278,457 to Campbell et al, simply warm this stream from the demethanizer in a heat exchanger before transporting it elsewhere.

Other patents, such as US-A-4,318,723 and US-A-4,350,511 to Holmes et al., not only warm this overhead vapour stream, but also condense a portion of it and return this condensed portion to the distillation column as reflux. Perhaps the most pertinent patent is US-A-4,687,499 to Aghili which first warms then compresses the overhead vapour stream before returning a portion of it back to the demethanizer as reflux, but only after this reflux portion has first been chilled and expanded.

While all of these processes are functional, only the Holmes et al. and Aghili patents recycle a portion of the removed vapour stream back to the column as a liquid. Holmes et al. condenses a portion of the vapour stream while Aghili's expansion of the chilled overhead vapour product causes some of this gas to condense into a liquid. The return of a liquid reflux is desirable because it is the condensed liquid that increases the recovery percentage of the desired column bottoms product. Analysis has shown that the reflux effect is optimized when the vapour recycle stream is totally condensed before expansion to the demethanizer operating pressure. Subcooling of the condensed reflux stream is usually less effective than increasing the rate of non-subcooled condensed liquid. Unfortunately however, a large portion of the reflux streams of Holmes et al. and Aghili are still vapour which does not increase product recovery. Instead, this uncondensed vapour mixes with the residue gas in the demethanizer and both are summarily discharged as the overhead vapour stream.

According to the invention there is provided a method of separating ethane from a natural gas stream wherein the natural gas stream is cooled and fed to a demethanizer and condensed liquid is returned to the demethanizer as reflux comprising the steps of:

a) removing overhead vapour from the demethanizer,

b) compressing only a portion of the overhead vapour, now reflux, to a pressure greater than that of the demethanizer,

c) reducing the temperature of the compressed reflux thereby causing it to condense, the temperature of the compressed reflux being reduced by cross-exchanging it with an uncompressed portion of the overhead vapour in a heat exchanger,

d) equalizing the pressure of the reflux to that of the demethanizer; and

e) feeding the reflux back to the demethanizer.

Thus a recycle stream containing liquid is returned to the top of a demethanizer for increased ethane recover in the column bottoms product. The recycle stream can be totally condensed thereby maximizing the recovery of ethane. The recycle stream equipment and hardware can be reduced in size yet provide the same amount of liquid reflux to the top of the demethanizer as is currently accomplished by Aghili or Holmes et al. Should the same size equipment be used for this process as is used for conventional processes, more liquid reflux can be delivered to the top of the demethanizer column. The number of expander-compressors and other equipment needed can be reduced since the recycle stream is fully condensed.

Chilling or cooling the reflux in a heat exchanger cases at least a portion of it but generally all of it to condense to liquid. After the pressure of this chilled and at least partially liquid stream is equalised to that of the demethanizer it can be fed or recycled back to the top of the demethanizer. The effect of this liquid (as compared to a vapour) in the demethanizer increases its operational efficiency and enhances the recovery of ethane from the demethanizer as a column bottoms product.

The invention is diagrammatically illustrated by way of example in the accompanying drawings, in which:-

Figure 1 is a schematic illustration of the method of improving ethane recovery from natural gas according to the invention showing in particular how a portion of an overhead vapour stream from a demethanizer is recycled back to the top of the demethanizer; and

Figure 2 is a schematic illustration similar to Figure 1 yet showing the process parameters resulting from an approximate 10% increase in overall power requirements along with a slight increase in the operating pressure of the demethanizer.

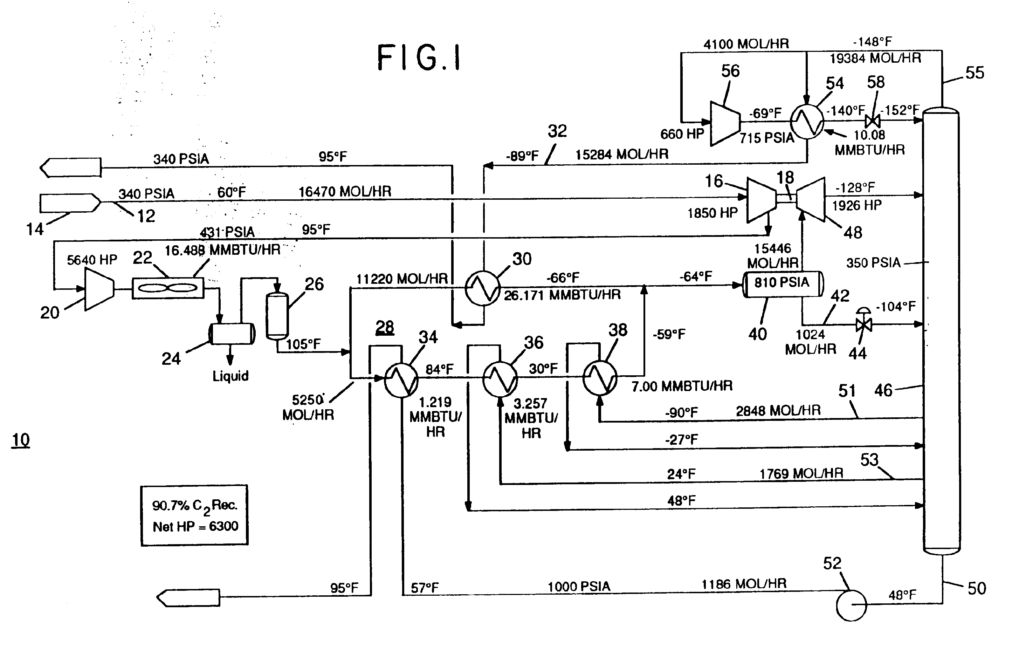

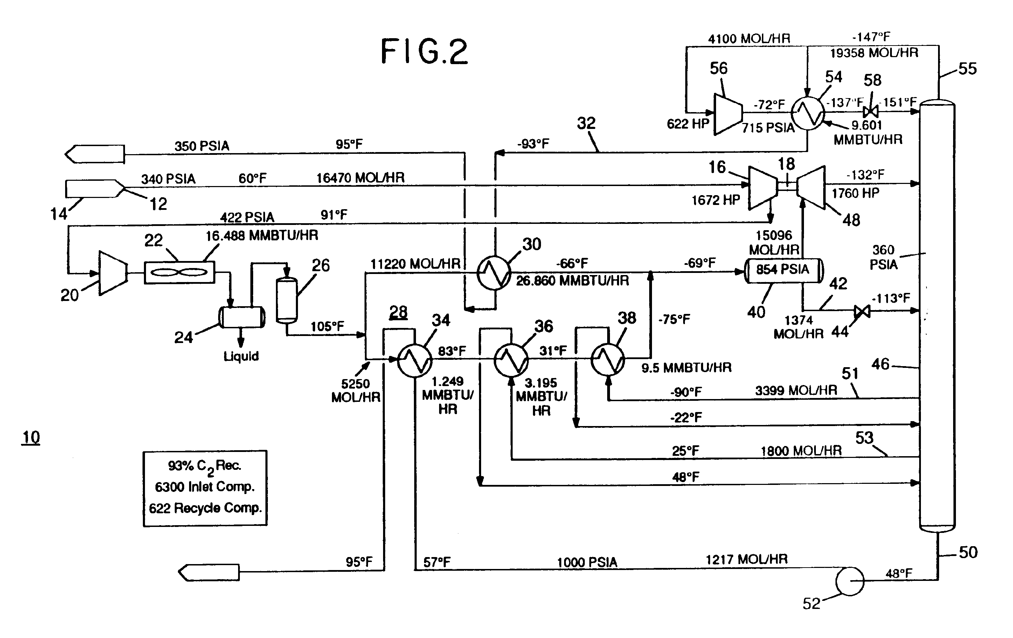

Referring to the drawings, there is shown a schematic illustration of ethane recovery process 10. Various values of temperature and pressure are recited throughout this illustration, but it should be emphasized that these are approximate values only and are recited solely as an aid in describing the process 10. There should be no mistaking these approximate values as the only values applicable. To illustrate this, Figure 2 is the same process as that shown in Figure 1 except that the cryogenic compressor and inlet compressor are operating at different power ratings. This change alone causes many different values of pressure and temperature to occur. With this understanding of the following pressure and temperature values being descriptive of only one of many variations of process 10, the intricacies of this process can be more fully described and understood.

A cleaned, filtered, and in all likelihood, dehydrated natural gas stream 12 enters ethane recovery process 10 through an inlet 14. In this embodiment and at this stage, the natural gas stream 12 will be supplied at the inlet 14 at a temperature of approximately 16°C (60°F) and at a pressure of about $2.34 \times 10^6$ Pa (340 psia). In this example, the molar flowrate of the stream 12 is taken to be 2075 MOL/S (gramme-mole/second)(16470 MOL/HR i.e. pound-mole/hour). The stream 12 flows into a compressor end 16 of an expander-compressor 18 where its pressure is increased to about $2.97 \times 10^6$ Pa (431 psia) and its temperature is raised to around 35°C (95°F). The anticipated rating of the compressor end 16 is approximately $13.8 \times 10^5$ watts (1850 H.P.). The gas stream 12 is then further compressed in an inlet gas compressor 20 driven by a gas turbine or other prime mover. In accordance with Figure 1, the inlet gas compressor 20 is operating at a horsepower rating of approximately $4.2 \times 10^6$ watts (5640 H.P.).

This highly pressurized gas stream is then cooled by cross exchange with air, water, or any other process by an inlet gas compressor discharge cooler 22. The rating of the compressor discharge cooler 22 for this example is $4.83 \times 10^6$ watts ($16.488 \times 10^6$ BTU/HR). Afterwards, the high pressure gas stream 12 is sent to a separator 24 where any liquid which may have formed in the previous compression or cooling steps is removed. The gas stream from the separator 24 is then dehydrated in desiccant bed driers or in any other suitable gas dehydrator 26. At this point, the temperature of the dehydrated natural gas is about 40.5°C (105°F) which is subsequently cooled in an inlet heat exchange train 28.

Cooling of the gas stream 12 is accomplished in the inlet heat exchange train 28 by splitting the stream and sending the major portion, approximately 2413.9 MOL/S (11220 MOL/HR) to a gas-gas 30 where it is cooled by cross exchange with a demethanizer overhead vapour stream 32. The rate of cooling of the gas-gas 30 is around $7.66 \times 10^6$ watts ($26.171 \times 10^6$ BTU/HR). After such cooling, this portion of the stream 12 is chilled to a temperature of about minus 54.4°C (minus 66°F). The remainder of the gas stream 12 about 661.3 MOL/S (about 5250 MOL/HR) is delivered, in series, to a demethanizer bottoms heater 34, demethanizer reboiler 36, and demethanizer side reboiler 38 heat exchangers. Each of these heat exchangers operates at a different cooling level with the demethanizer bottoms heater 34 operating at approximately $3.57 \times 10^5$ watts ($1.219 \times 10^6$ BTU/HR), the demethanizer reboiler 36 operating at approximately $9.54 \times 10^5$ watts ($3.257 \times 10^6$ BTU/HR), and the demethanizer side reboiler 38 operating at approximately $2.05 \times 10^6$ watts ($7.00 \times 10^6$ BTU/HR). After the first cross exchange in the bottoms heater 34, the temperature of this portion of the gas stream 12 is about 289°C (84°F), after the reboiler 36, the temperature is approximately minus 1°C (30°F) and after the side reboiler 38 the temperature is about minus 50.5°C (minus 59°F). Upon combining the previously separated streams, the temperature of the mixture settles around minus 53.3°C (minus 64°F). It should be noted that additional refrigeration of the gas stream may occur either while it is separated or after being combined, as needed.

The combined cooled inlet stream then flows to a cold separator 40 which generally is at a pressure of about $5.5 \times 10^6$ Pa (810 psia). Here, any condensed liquid is separated from the uncondensed gas stream. A line 42 transfers this condensed liquid, having a flow rate of about 129.0 MOL/S (1024 MOL/HR), across a cold separator liquid valve 44 and to a lower feed tray of a demethanizer 46. At this lower feed inlet, the liquid stream in the line 42 has a temperature of about minus 75.5°C (minus 104°F) and its pressure, which has been reduced via the valve 44, is approximately that of the demethanizer, here operating at approximately $2.4 \times 10^6$ Pa (350 psia).

Vapour from the cold separator 40 having a flow rate of approximately 1946.4 MOL/S (15446 MOL/HR) flows through an expander end 48 of the expander-compressor 18 where its pressure is reduced from the $5.58 \times 10^6$ Pa (810 psia) found in the cold separator 40 to that of the demethanizer 46 approximately $2.4 \times 10^6$ Pa (350 psia). The horsepower rating of the expander end 48 is approximately $14.37 \times 10^5$ Watts (1926 H.P.) and the expander-compressor 18 lowers the temperature of this vapour stream to about minus 88.9°C (minus 128°F) before it is fed to a middle feed location of the demethanizer 46.

Within the demethanizer 46, methane and lighter, more volatile constituents of the natural gas stream 12 vapourise and separate from such heavier counterparts as ethane, propane and butane. Ethane and these heavier constituents are recovered as the demethanizer column bottoms product and are removed from the demethanizer 48 via a line 50. Before being recovered, the ethane is warmed to a temperature of about 8.9°C (48°F) after being cross-exchanged with the inlet natural gas stream in the reboiler 36 and the side reboiler 38 heat exchangers. As shown in Figure 1, a line 51 transfers a portion of the flow, (approximately 358.8 MOL/S (2848 MOL/HR), in the demethanizer 46 to and from the side reboiler heat exchanger 38. This flow is initially at a temperature of about minus 67.8°C (minus 90°F) before it is crows exchanged with the gas stream 12 at a temperature of about minus 1°C (30°F). After such exchange, the gas stream 12 is chilled to about minus 50.5°C (minus 59°F) while the line 51 is warmed to about minus 32.7°C (minus 27°F).

Likewise, a line 53 transmits about 222.9 MOL/S (1769 MOL/HR) from the demethanizer 46 for cross exchange in the reboiler heat exchanger 36. Prior to such heat exchange, the line 53 is at a temperature of about minus 4.4°C (24°F) whereas after such heat exchange in the reboiler 36, its temperature rises to approximately 8.9°C (48°F). At the same time the cross exchanging fluid stream 12, has its temperature reduced from approximately 28.9°C (84°F) to about minus 1°C (30°F).

As indicated earlier, the demethanizer bottoms product leaves the demethanizer 46 via the line 50 at a temperature of about 8.9°C (48°F). Upon departure, a demethanizer pump 52 pressurizes this ethane and other heavier components to a pressure of about 6.8 x 10$^6$Pa (1000 psia) causing its temperature to increase to about 13.8°C (57°). After such pressurization, this liquid, having a flow rate of about 149.4 MOL/S (1186 MOL/HR) flows through the bottoms heater 34 where it is cross exchanged with the gas stream 12. In the bottoms heater 34, the demethanizer bottoms product has its temperature raised from approximately 13.8°C (57°F) to about 35°C (95°F) whereas the gas stream 12 is chilled from approximately 40.6°C (105°F) to about 28.9°C (84°F). Afterwards, the ethane and other heavier constituents of the natural gas stream are transported elsewhere for further processing.

The lighter overhead methane gas stream exits the top of the demethanizer 46 via a line 55 at a temperature of about minus 100°C (minus 148°F). This overhead vapour stream is split into two streams, the majority, approximately 1925.8 MOL/S (15284 MOL/HR), flows first through a recycle exchanger 54 and then through the gas-gas exchanger 30 before being transported elsewhere. The recycle exchanger 54 raises the temperature of this majority portion of the line 55 from approximately minus 100°C (minus 148°F) to about minus 67.2°C (minus 89°F) while the gas-gas exchanger 30, which is in cross exchange with the incoming gas stream 12, raises its temperature to approximately 35°C (95°F). At this temperature, and at a pressure of about 2.3 x 10$^6$Pa (340 psia), the majority of the overhead vapour stream (i.e., the lighter methane and other more volatile components) is transported elsewhere for further processing.

A smaller portion of this overhead methane gas stream or reflux approximately 516.7 MOL/S (4100 MOL/HR) is directed to a cryogenic compressor 56. The compressor 56, which is rated at about 4.9 x 10$^5$ Watts (660 HP), increases the pressure of this minority portion of the line 55 from approximately 2.4 x 10$^6$Pa (350 psia) (the pressure of the demethanizer 46) to about 4.9 x 10$^6$Pa (715 psia). In doing so, the temperature of this reflux stream is raised from approximately minus 100°C (minus 148°F) to about minus 56°C (minus 69°F). This compressed methane gas reflux is then condensed by cross-exchange with the majority of the overhead methane gas stream in the recycle exchanger 54. The capacity of the recycle exchanger 54 is about 29.5 x 10$^5$ Watts (10.08 x 10$^6$BTU/HR) and such cross-exchange lowers the temperature of the reflux to about minus 95.5°C (minus 140°F). The compressed reflux leaving the recycle exchanger 54 may be partially condensed, totally condensed, or totally condensed and subcooled in the exchanger 54 depending on the parameters chosen by the operator. The parameters described herein will provide a totally condensed stream. No matter its state, this reflux stream passes through a recycle valve 58 which reduces its pressure to that of the demethanizer 46 approximately 2.4 x 10$^6$Pa (350 psia) while also lowering its temperature to about minus 102.2°C (minus 152°F). This compressed, cooled, and condensed reflux, which initially formed a portion of the overhead methane gas stream 55, is then recycled back to the top of the demethanizer 46 where the condensed liquid increases the efficiency and ability of the demethanizer 46 to separate methane from the other natural gas components thereby producing more ethane as a column bottoms product.

Referring now more specifically to Figure 2, the same process is followed with the differences being an approximate 10% increase in the overall power requirement of the ethane recovery process 10 and a change in the operating pressure of the demethanizer 46. The power supplied to the cryogenic compressor 56 in Figure 2 is actually reduced about 10% from the process shown in Figure 1, from approximately 4.9 x 10$^5$ watts (660 HP) in Figure 1 to about 4.5 x 10$^5$ watts (622 H.P.) in Figure 2. Furthermore, the power supplied to the inlet gas compressor 20 is increased about 10% over that disclosed in Figure 1, from approximately 4.2 x 10$^6$ watts (5640 HP) to about 4.7 x 10$^6$ watts (6300 HP). With these changes, the pressure in the cold separator 40 is raised from approximately 5.5 x 10$^6$Pa (810 psia) to about 5.9 x 10$^6$Pa (854 psia). Also, the pressure in the demethanizer 46 is raised from approximately 2.4 x $10^6$Pa (350 psia) to about 2.5 x $10^6$Pa (360 psia). These changes have the specific effect of altering certain pressure and temperature values of the process 10 with the overall effect being to increase the efficiency of ethane recovery from about 90.7% (Figure 1) to about 93% (Figure 2).

In natural gas streams containing carbon dioxide, the proposed process achieves higher levels of ethane recovery than a conventional process. This is because for a given ethane recovery percentage, the proposed process allows the demethanizer to operate at a temperature and pressure higher than that of a conventional process. Such higher operating temperature and pressure places the system operating conditions further away from the conditions under which solid carbon dioxide forms. Now, if the operating conditions of the proposed process are moved closer to the solid carbon dioxide formation conditions, ethane recovery will increase even more and, likewise, operating power will increase. Depending on the carbon dioxide content of a particular feed gas stream, the power requirements, if desired, may be increased up to the level of the conventional process, or the operating conditions may maintain a critical distance from solid carbon dioxide formation conditions. Nevertheless, all in all, the ethane recovery will be higher and the operating power will be lower than for a conventional process.

Another feature of the ethane recovery process 10 is that a recycle stream which is totally condensed can be supplied to the demethanizer 46 to maximize ethane recovery. This can be accomplished by proper control of the interrelationship between the cryogenic compressor 56, the recycle exchanger 54, the recycle valve 58 and the operating pressure of the demethanizer 46. Consequently, should a user desire a certain amount of liquid reflux, the above equipment can be installed for just that amount so that oversized or undersized equipment can be avoided. Additionally, due to the fact that the reflux is all liquid, a reflux expander-compressor is not necessary. The power recovery and cooling obtained from a liquid expander is not significant enough to be economical. Simply reducing the condensed reflux stream's pressure across a valve adequately satisfies the process requirements.

Finally, the decoupling or separation of the above recycle/reflux scheme from the inlet supply process makes each process separately controllable. It is a disadvantage to have these schemes overlap because in doing so it becomes very difficult, if not impossible, to operate the process 10 should something be wrong with the reflux system. By decoupling them, the reflux system can be by-passed, if need be, without affecting the inlet gas stream 12 or the operation of process 10 in the conventional manner.

In view of the above embodiment, it should be noted that more compression activity takes place on the inlet side of the demethanizer 46 as contrasted with the discharge or residue gas side of the demethanizer 46. One basic variation which is not shown in the drawings is to reverse this and shift the main compression duty from the inlet gas stream to the residue gas stream. This variation is needed when the inlet natural gas stream 12 is available at a high pressure and the overhead vapour stream 32 (the residue gas stream) must also be delivered at a high pressure. To accomplish this, the compressor end 16 of the expander-compressor 18 would be relocated in the process 10 so as to compress the residue gas stream 32 after it is warmed in the gas-gas exchanger 30. The warmed gas leaving the expander-compressor 18 would normally then be further compressed by a residue gas compressor which is driven by an electric motor, gas engine, turbine, or other prime mover. Such high pressure gas would then be cooled by cross exchange with air, water, or any other available process or utility stream. This cooled, high pressure gas is then delivered as the residue gas product stream.

## Claims

1. A method of separating ethane from a natural gas stream (12) wherein the natural gas stream is cooled and fed to a demethanizer (40) and condensed liquid is returned to the demethanizer (46) as reflux comprising the steps of:

   a) removing overhead vapour from the demethanizer (46),

   b) compressing only a portion of the overhead vapour, now reflux, to a pressure greater than that of the demethanizer (46),

   c) reducing the temperature of the compressed reflux thereby causing it to condense, the temperature of the compressed reflux being reduced by cross-exchanging it with an uncompressed portion of the overhead vapour in a heat exchanger (54),

   d) equalizing the pressure of the reflux to that of the demethanizer (54); and

   e) feeding the reflux back to the demethanizer.

2. A method according to claim 1, wherein the reflux is totally condensed before being fed to the demethanizer (46).

3. A method according to claim 2, wherein the reflux is compressed in a cryogenic compressor (56).

4. A method according to claim 3, wherein the pressure of the reflux is equalized to that of the demethanizer (46) across a recycle valve (58).

## Patentansprüche

1. Verfahren zur Abtrennung von Ethan von einem Naturgasstrom (12), bei dem der Naturgasstrom gekühlt und in eine Methanentfernervorrichtung (46) eingespeist und bei dem kondensierte Flüssigkeit zu der Methanentfernervorrichtung (46) als Rückfluß zurückgeführt wird, mit den Stufen, in denen man

a) Kopfdampf von der Methanentfernervorrichtung (46) entfernt,

b) nur einen Teild es Kopfdampfes, nun Rückfluß, bis zu einem Druck größer als der der Methanentfernervorrichtung (46) komprimiert,

c) die Temperatur des komprimierten Rückflusses vermindert und so seine Kondensation bewirkt, wobei die Temperatur des komprimierten Rückflusses durch seinen gegenseitigen Austausch mit einem unkomprimierten Teil des Kopfdampfes in einem Wärmeaustauscher (54) vermindert wird,

d) den Druck des Rückflusses mit dem der Methanentfernervorrichtung (46) gleichmacht und

e) den Rückfluß zurück zu der Methanentfernervorrichtung führt.

2. Verfahren nach Anspruch 1, bei dem man den Rückfluß vollständig kondensiert, bevor er zu der Methanentfernervorrichtung (46) geführt wird.

3. Verfahren nach Anspruch 1, bei dem man den Rückfluß in einem kryogenen Kompressor (56) komprimiert.

4. Verfahren nach Anspruch 3, bei dem man den Druck des Rückflusses mit dem der Methanentfernervorrichtung (46) in einem Kreislaufventil (58) gleichmacht.

## Revendications

1. Procédé de séparation d'éthane dans un flux de gaz naturel (12), dans lequel le flux de gaz naturel est refroidi et amené dans une colonne de séparation de méthane (40) et le liquide condensé est ramené dans la colonne de séparation de méthane (46) sous forme de produit de reflux, comprenant les opérations consistant à :

a) éliminer la vapeur de tête de la colonne de séparation de méthane (46),

b) comprimer seulement une partie de la vapeur de tête, maintenant sous forme de produit de reflux, à une pression supérieure à celle de la colonne de séparation de méthane (46),

c) réduire la température du produit de reflux comprimé, ce qui provoque sa condensation, la température du produit de reflux comprimé étant réduite par un échange croisé de celui-ci avec une partie non comprimée de la vapeur de tête dans un échangeur de chaleur (54),

d) amener la pression du produit de reflux à la pression de la colonne de séparation de méthane (46); et

e) ramener le produit de reflux dans la colonne de séparation de méthane.

2. Procédé selon la revendication 1, dans lequel le produit de reflux est complètement condensé avant d'être amené dans la colonne de séparation de méthane (46).

3. Procédé selon la revendication 2, dans lequel le produit de reflux est comprimé dans un compresseur cryogénique (56).

4. Procédé selon la revendication 3, dans lequel on égalise la pression du produit de reflux par rapport à celle de la colonne de séparation de méthane (46), à travers une soupape de recyclage (58).

FIG.1

90.7% C₂Rec.
Net HP = 6300

EP 0 370 611 B1

# FIG.2

EP 0 370 611 B1

4100 MOL/HR    -147°F

19358 MOL/HR

55

56    54    58

-72°F    -137°F   -151°F

622 HP    715 PSIA    9.601 MMBTU/HR

350 PSIA    95°F    -93°F    32

340 PSIA    60°F    16470 MOL/HR

16   18    -132°F

1672 HP    1760 HP

14    12    48

422 PSIA    91°F

22    16.488 MMBTU/HR    30    15096 MOL/HR    360 PSIA

11220 MOL/HR    -66°F    -69°F

26    854 PSIA    42

20    26.860 MMBTU/HR    40    -113°F

24    105°F    28   34    36    38    -75°F    1374 MOL/HR   44

Liquid    83°F    31°F    9.5 MMBTU/HR    46

5250 MOL/HR    1.249 MMBTU/HR    3.195 MMBTU/HR    -90°F    3399 MOL/HR   51

-22°F

10    25°F    1800 MOL/HR   53

93% C$_2$ Rec.    48°F

6300 Inlet Comp.

622 Recycle Comp.

50

95°F    57°F    1000 PSIA    1217 MOL/HR    48°F

52

8